Europäisches Patentamt

(19) European Patent Office

Office européen des brevets

(11) Publication number : 0 431 759 B1

# EUROPEAN PATENT SPECIFICATION

(45) Date of publication of patent specification :
12.01.94 Bulletin 94/02

(51) Int. Cl.⁵ : **A61K 9/18, A61K 31/34**

(21) Application number : **90312263.8**

(22) Date of filing : **09.11.90**

(54) Process for the preparation of a ranitidine resin absorbate.

The file contains technical information submitted after the application was filed and not included in this specification

(30) Priority : **10.11.89 GB 8925484**

(43) Date of publication of application :
**12.06.91 Bulletin 91/24**

(45) Publication of the grant of the patent :
**12.01.94 Bulletin 94/02**

(84) Designated Contracting States :
**AT BE CH DE DK ES FR GB GR IT LI LU NL SE**

(56) References cited :
**DE-A- 2 246 037**
**FR-A- 1 335 502**
**GB-A- 1 462 356**
**GB-A- 2 198 352**
**GB-A- 2 218 333**
**US-A- 3 342 685**
**PATENT ABSTRACTS OF JAPAN, vol. 13, no. 579 (C-668)[3927], 20th December 1989; & JP-A-1 242 522 (FUJISAWA PHARMACEUTI-CAL) 27-09-1989**

(56) References cited :
**JOURNAL OF PHARMACEUTICAL SCIENCES, vol. 60, no. 10, 1971, pages 1523-1527; S. BORODKIN et al.: "Polycarboxylic acid ion-exchange resin adsorbates for taste coverage in chewable tablets"**
**DERWENT FILE SUPPLIER WPIL, 1990, AN=90-009109 [02], Derwent publications Ltd, London, GB; & EP-A-349 103 (03-01-1990) & AU-A-3 394 689 (09-11-1989) (SMITH KLINE FRENCH)**

(73) Proprietor : **GLAXO GROUP LIMITED**
**Clarges House 6-12 Clarges Street**
**London W1Y 8DH (GB)**

(72) Inventor : **Douglas, Stephen John, Glaxo Group Research Ltd.**
**Park Road, Ware**
**Hertfordshire, SG12 0DG (GB)**
Inventor : **Bird, Fiona Ruth, Glaxo Group Research Ltd.**
**Park Road, Ware**
**Hertfordshire, SG12 0DG (GB)**

(74) Representative : **Marchant, James Ian Elkington and Fife Prospect House 8 Pembroke Road**
**Sevenoaks, Kent TN13 1XR (GB)**

## Description

The present invention relates to a process for the preparation of a drug adsorbate, more particularly a drug adsorbate in which the active ingredient is ranitidine.

In our published U.K. Patent Specification No. 2218333A, which was unpublished at the priority date of the present application, we disclose resin adsorbates of ranitidine which comprise complexes formed between ranitidine and a synthetic cation exchange resin. The formation of such adsorbates provides a means of masking the bitter taste associated with ranitidine.

One of the methods described in U.K. Patent Specification No. 2218333A for the preparation of the adsorbates involves mixing the synthetic cation exchange resin with ranitidine or a physiologically acceptable salt thereof in a solvent such as water. We have now found that it is particularly advantageous to carry out this process in an alcoholic solvent.

DE-A-2246037 relates to delayed-release medicaments comprising film-coated resin adsorbate particles. The formation of resinates in the presence of an alcohol as solvent is discussed but in the context of the speed of absorption of the medicament on to the resin and the degree of loading on the resin. Neither $H_2$-antagonists as a class nor ranitidine in particular are mentioned as possible active substances for the formation of resinates so that there is no teaching of any possible process for the preparation of such a resinate and no indication of its likely stability.

The present invention thus provides a process for the preparation of a resin adsorbate of ranitidine which comprises contacting a synthetic cation exchange resin with ranitidine or a physiologically acceptable salt thereof in the presence of an alcoholic solvent.

The ranitidine resin adsorbate prepared according to the process of the invention has a low water content, typically less than 2-3% on a weight-to-weight basis, and the product thus obtained is significantly more stable than that resulting from the use of water as solvent. This in turn leads to a product possessing an improved shelf life.

According to a preferred embodiment of the process of the invention, the resin adsorbate may be prepared by mixing the synthetic cation exchange resin with a solution of ranitidine in the form of either its free base or a physiologically acceptable salt, in the alcoholic solvent.

The alcoholic solvent may be for example a lower ($C_{1-4}$) alkanol, more preferably methanol, ethanol or mixtures thereof. The use of a mixture of methanol and ethanol is particularly preferred.

The process may conveniently be carried out at a temperature between room temperature and the reflux temperature of the alcoholic solvent. A temperature within the range $50^0$ to $70^0C$ is preferred.

The synthetic cation exchange resin for use in the process of the invention may be for example a copolymer of styrene or acrylic or methacrylic acid with a vinyl aromatic compound such as divinylbenzene, and the resin may derive its exchange activity from either weakly or strongly acidic groups such as carboxylic acid or sulphonic acid groups. Examples of suitable resins are those that are copolymers of styrene and divinylbenzene which are sulphonated, or copolymers of methacrylic acid and divinylbenzene, including those available commercially as Dowex resins (available from Dow Chemical Company) or Amberlite resins (available from Rohm & Haas). The use of copolymers of methacrylic acid and divinylbenzene is preferred.

The resin may be in the form of a salt with an alkali metal (e.g. sodium or potassium) or, more preferably, in free acid form.

The resins used should be non-toxic and pharmaceutically acceptable.

Ranitidine may be employed in the process according to the invention in the form of either its free base or a physiologically acceptable salt. Use of the free base is preferred. When a salt is used this may be a salt with an inorganic or organic acid such as a hydrochloride, hydrobromide, sulphate, acetate, maleate, succinate, fumarate or ascorbate salt, of which the hydrochloride is preferred.

According to a particularly preferred embodiment of the process according to the invention, ranitidine in the form of its free base is contacted with a methacrylic acid-divinylbenzene resin in free acid form (e.g. Amberlite IRP-64).

The ranitidine content of the resin adsorbate prepared according to the process of the invention may be in the range of for example 5% to 70% on a weight-to-weight basis, expressed in terms of the weight of ranitidine free base. The ranitidine content of the adsorbate is preferably 15% to 55%, more preferably 25% to 35%.

According to a further aspect, the invention provides a resin adsorbate formed between ranitidine and a synthetic cation exchange resin, characterised in that the water content of the resin adsorbate is less than 3% on a weight-to weight (w/w) basis, more particularly less than 2% w/w, and preferably less than 1% w/w.

According to a preferred embodiment of this further aspect of the invention, the resin adsorbate is formed between ranitidine and a methacrylic acid - divinylbenzene resin in free acid form (e.g. Amberlite IRP-64).

According to yet another aspect, the invention provides a pharmaceutical composition, for oral use in human or veterinary medicine, containing a resin adsorbate formed between ranitidine and a synthetic cation exchange resin, particularly a methacrylic acid - divinylbenzene resin in free acid form (e.g. Amberlite IRP-64), characterised in that the water content of the resin adsorbate is less than 3% w/w, more particularly less than 2% w/w, and preferably less than 1% w/w.

The compositions according to the invention may for example take the form of tablets, capsules, granules, powders, tablets or lozenges for buccal administration, or liquid preparations such as suspensions. Granules and powders may be ingested directly, or dispersed in water or other suitable vehicle prior to administration. Capsules may be of the hard or soft gelatin type, including chewable soft gelatin capsules.

Chewable or suckable tablets (including cast chewable tablets), chewable soft gelatin capsules, granules and non-aqueous suspensions represent preferred dosage forms.

The compositions may be formulated using conventional carriers or excipients and well established techniques, as described in for example published U.K. Patent Specification No. 2218333A.

The following Examples illustrate the invention. Temperatures are in degrees Centigrade. Water content was determined by coulometric Karl Fischer titration.

## Example 1

Amberlite IRP-64 (13.7kg) was suspended in industrial methylated spirits B.P. (IMS B.P.) (75 l) and heated to reflux. To this stirred refluxing suspension was added a solution of ranitidine free base (6.3kg) in IMS B.P. (25 l). The mixture was then stirred under reflux for one hour before cooling to room temperature. The solid was filtered off, washed with IMS B.P. (3x20 l) and then dried under vacuum at $50^0$ to constant weight. The resulting product was a white to off-white powder containing 30.2% w/w ranitidine base equivalent in which the bitter taste of the drug was substantially reduced. Water content of the product was 0.4% w/w.

## Example 2

To a stirred suspension of Amberlite IRP-64 (22.2g) in methanol (70ml) was added a solution of ranitidine free base (7.8g) in methanol (30ml). The mixture was stirred at room temperature for three hours. The solid was isolated by filtration, washed with methanol (3 x 30ml) and dried under vacuum at $50^0$ to constant weight. The resulting product was an off-white powder containing 24.2% w/w ranitidine base equivalent in which the bitter taste of the drug was substantially reduced.

## Example 3

To a solution of ranitidine free base (3g) in absolute ethanol (50ml) was added Amberlite IRP-64 (7g). This mixture was stirred under reflux for one hour and then cooled to room temperature. The solid was filtered off, washed with absolute ethanol (3 x 20ml) and dried under vacuum at $50^0$. The resulting product was an off-white powder containing 27.2% w/w ranitidine base equivalent in which the bitter taste of the drug was substantially reduced. Water content of the product was 0.9% w/w.

## Example 4

A solution of ranitidine free base (20.0g) in IMS (40ml) at $60^0$ was added to a stirred suspension of Amberlite IRP-64 (43.4g) in IMS (182ml) at $60^0$. The resulting suspension was stirred at $60^0$ for two hours, and then cooled gradually with stirring to room temperature. The solid was collected by filtration, washed with IMS (3 x 64ml) and dried under vacuum at $50^0$. The resulting product (63.2g) contained approx. 30% w/w ranitidine base equivalent.

The following Example A illustrates a pharmaceutical composition in which the drug resinate is in particular a ranitidine-Amberlite IRP-64 resinate having low water content and prepared according to the process of the invention.

Example A Non-Aqueous Suspension

|  | % w/v |
|---|---|
| Drug resinate* | 10.0 |
| Xylitol | 20.0 |
| Aspartame | 0.4 |
| Colloidal silica | 0.3 |
| Citric acid | 0.1 |
| Flavour | 0.2 |
| Fractionated coconut oil to | 100.0 |

*containing 30% w/w ranitidine base equivalent

The drug resinate and xylitol are dispersed in the bulk of the fractionated coconut oil by high shear mixing. The remaining ingredients are added and mixed in using a suitable mixer. The suspension is made up to volume with fractionated coconut oil and mixed well. Each 5ml of suspension contains 150mg ranitidine base equivalent.

## Claims

**Claims for the following Contracting States : AT, BE, CH, DE, DK, FR, GB, IT, LI, LU, NL, SE**

1. A process for the preparation of a resin adsorbate of ranitidine which comprises contacting a synthetic cation exchange resin with ranitidine or a physiologically acceptable salt thereof in the presence of an alcoholic solvent.

2. A process as claimed in Claim 1 wherein the synthetic cation exchange resin is mixed with a solution of ranitidine in the form of the free base or a physiologically acceptable salt thereof in the alcoholic solvent.

3. A process as claimed in Claim 1 or 2 wherein the alcoholic solvent is methanol, ethanol or a mixture thereof.

4. A process as claimed in any of Claims 1 to 3 carried out at a temperature from room temperature to the reflux temperature of the alcoholic solvent.

5. A process as claimed in Claim 4 carried out at a temperature from 50° to 70°C.

6. A process as claimed in any of Claims 1 to 5 wherein the synthetic cation exchange resin is a copolymer of methacrylic acid and divinylbenzene in free acid form.

7. A process as claimed in any of Claims 1 to 6 wherein the ranitidine is used in the form of the free base.

8. A process as claimed in any of Claims 1 to 7 wherein the cation exchange resin and the ranitidine are used in amounts such that the resin adsorbate has a ranitidine content of from 5% to 70% on a weight-to-weight basis expressed as the weight of ranitidine free base.

9. A process as claimed in Claim 8 wherein the ranitidine content of the resin adsorbate is 25% to 35%.

4

10. A resin adsorbate formed between ranitidine and a synthetic cation exchange resin having a water content of less than 3% on a weight-to-weight basis.

11. A resin adsorbate as claimed in Claim 10 wherein the water content is less than 1%.

12. A resin adsorbate as claimed in Claim 10 or 11 formed between ranitidine and a methacrylic acid-divinylbenzene resin in free acid form.

13. A pharmaceutical composition for oral use in human or veterinary medicine containing a resin adsorbate when prepared by a process as claimed in any of Claims 1 to 9.

14. A pharmaceutical composition for oral use in human or veterinary medicine containing a resin adsorbate as claimed in any of Claims 10 to 12.

**Claims for the following Contracting States : ES, GR**

1. A process for the preparation of a resin adsorbate of ranitidine which comprises contacting a synthetic cation exchange resin with ranitidine or a physiologically acceptable salt thereof in the presence of an alcoholic solvent.

2. A process as claimed in Claim 1 wherein the synthetic cation exchange resin is mixed with a solution of ranitidine in the form of the free base or a physiologically acceptable salt thereof in the alcoholic solvent.

3. A process as claimed in Claim 1 or 2 wherein the alcoholic solvent is methanol, ethanol or a mixture thereof.

4. A process as claimed in any of Claims 1 to 3 carried out at a temperature from room temperature to the reflux temperature of the alcoholic solvent.

5. A process as claimed in Claim 4 carried out at a temperature from 50° to 70°C.

6. A process as claimed in any of Claims 1 to 5 wherein the synthetic cation exchange resin is a copolymer of methacrylic acid and divinylbenzene in free acid form.

7. A process as claimed in any of Claims 1 to 6 wherein the ranitidine is used in the form of the free base.

8. A process as claimed in any of Claims 1 to 7 wherein the cation exchange resin and the ranitidine are used in amounts such that the resin adsorbate has a ranitidine content of from 5% to 70% on a weight-to-weight basis expressed as the weight of ranitidine free base.

9. A process as claimed in Claim 8 wherein the ranitidine content of the resin adsorbate is 25% to 35%.

**Patentansprüche**

**Patentansprüche für folgende Vertragsstaaten : AT, BE, CH, DE, DK, FR, GB, IT, LI, LU, NL, SE**

1. Verfahren zur Herstellung eines Harzadsorbats des Ranitidins, dadurch **gekennzeichnet,** daß ein synthetisches Kationenaustauschharz mit Ranitidin oder einem physiologisch annehmbaren Salz davon in Anwesenheit eines alkoholischen Lösungsmittels behandelt wird.

2. Verfahren nach Anspruch 1, dadurch **gekennzeichnet,** daß das synthetische Kationenaustauschharz mit einer Ranitidinlösung in Form der freien Base oder eines physiologisch annehmbaren Salzes davon in einem alkoholischen Lösungsmittel vermischt wird.

3. Verfahren nach Anspruch 1 oder 2, dadurch **gekennzeichnet,** daß als alkoholisches Lösungsmittel Methanol, Ethanol oder ein Gemisch davon verwendet wird.

4. Verfahren nach einem der Ansprüche 1 bis 3, dadurch **gekennzeichnet,** daß es bei einer Temperatur von Raumtemperatur bis zur Rückflußtemperatur des alkoholischen Lösungsmittels durchgeführt wird.

5. Verfahren nach Anspruch 4, dadurch **gekennzeichnet,** daß es bei einer Temperatur von 50 bis 70°C durchgeführt wird.

6. Verfahren nach einem der Ansprüche 1 bis 5, dadurch **gekennzeichnet,** daß als synthetisches Kationenaustauschharz ein Copolymeres aus Methacrylsäure und Divinylbenzol in freier Säureform verwendet wird.

7. Verfahren nach einem der Ansprüche 1 bis 6, dadurch **gekennzeichnet,** daß das Ranitidin in Form der freien Base verwendet wird.

8. Verfahren nach einem der Ansprüche 1 bis 7, dadurch **gekennzeichnet,** daß das Kationenaustauschharz und das Ranitidin in solchen Mengen verwendet werden, daß das Harzadsorbat einen Ranitidingehalt von 5 bis 70% auf Gewicht-zu-Gewicht-Basis, ausgedrückt als das Gewicht der freien Ranitidinbase, besitzt.

9. Verfahren nach Anspruch 8, dadurch **gekennzeichnet,** daß der Ranitidingehalt des Harzadsorbats 25 bis 35% beträgt.

10. Harzadsorbat, das zwischen Ranitidin und einem synthetischen Kationenaustauschharz gebildet worden ist und einen Wassergehalt von weniger als 3% auf Gewicht-zu-Gewicht-Basis besitzt.

11. Harzadsorbat nach Anspruch 10, dadurch **gekennzeichnet,** daß der Wassergehalt geringer ist als 1%.

12. Harzadsorbat nach Anspruch 10 oder 11, dadurch **gekennzeichnet,** daß es zwischen Ranitidin und einem Methacrylsäure-Divinylbenzol-Harz in freier Säureform gebildet worden ist.

13. Pharmazeutische Zusammensetzung für die orale Verwendung in der Human- oder Veterinärmedizin, dadurch **gekennzeichnet,** daß es ein Harzadsorbat, hergestellt nach einem Verfahren nach einem der Ansprüche 1 bis 9, enthält.

14. Pharmazeutische Zusammensetzung für die orale Verwendung in der Human- oder Veterinärmedizin, dadurch **gekennzeichnet,** daß sie ein Harzadsorbat nach einem der Ansprüche 10 bis 12 enthält.

**Patentansprüche für folgende Vertragsstaaten : ES, GR**

1. Verfahren zur Herstellung eines Harzadsorbats des Ranitidins, dadurch **gekennzeichnet,** daß ein synthetisches Kationenaustauschharz mit Ranitidin oder einem physiologisch annehmbaren Salz davon in Anwesenheit eines alkoholischen Lösungsmittels behandelt wird.

2. Verfahren nach Anspruch 1, dadurch **gekennzeichnet,** daß das synthetische Kationenaustauschharz mit einer Ranitidinlösung in Form der freien Base oder eines physiologisch annehmbaren Salzes davon in einem alkoholischen Lösungsmittel vermischt wird.

3. Verfahren nach Anspruch 1 oder 2, dadurch **gekennzeichnet,** daß als alkoholisches Lösungsmittel Methanol, Ethanol oder ein Gemisch davon verwendet wird.

4. Verfahren nach einem der Ansprüche 1 bis 3, dadurch **gekennzeichnet,** daß es bei einer Temperatur von Raumtemperatur bis zur Rückflußtemperatur des alkoholischen Lösungsmittels durchgeführt wird.

5. Verfahren nach Anspruch 4, dadurch **gekennzeichnet,** daß es bei einer Temperatur von 50 bis 70°C durchgeführt wird.

6. Verfahren nach einem der Ansprüche 1 bis 5, dadurch **gekennzeichnet,** daß als synthetisches Kationenaustauschharz ein Copolymeres aus Methacrylsäure und Divinylbenzol in freier Säureform verwendet wird.

7. Verfahren nach einem der Ansprüche 1 bis 6, dadurch **gekennzeichnet,** daß das Ranitidin in Form der freien Base verwendet wird.

8. Verfahren nach einem der Ansprüche 1 bis 7, dadurch **gekennzeichnet,** daß das Kationenaustauschharz und das Ranitidin in solchen Mengen verwendet werden, daß das Harzadsorbat einen Ranitidingehalt von 5 bis 70% auf Gewicht-zu-Gewicht-Basis, ausgedrückt als das Gewicht der freien Ranitidinbase, besitzt.

9. Verfahren nach Anspruch 8, dadurch **gekennzeichnet,** daß der Ranitidingehalt des Harzadsorbats 25 bis 35% beträgt.

**Revendications**

**Revendications pour les Etats contractants suivants : AT, BE, CH, DE, DK, FR, GB, IT, LI, LU, NL, SE**

1. Un procédé de préparation d'absorbate de résine de ranitidine comprenant le fait de mettre une résine synthétique à échange de cations en contact avec de la ranitidine ou un sel de celle-ci acceptable d'un point de vue physiologique, en présence d'un solvant alcoolique.

2. Un procédé selon la Revendication 1, dans lequel la résine synthétique à échange de cations est mélangée avec une solution de ranitidine sous la forme de sa base libre ou d'un sel de celle-ci acceptable d'un point de vue physiologique, dans le solvant alcoolique.

3. Un procédé selon la Revendication 1 ou 2, dans lequel le solvant alcoolique est du méthanol, de l'éthanol ou un mélange de ceux-ci.

4. Un procédé selon l'une des Revendications 1 à 3, effectué à une température comprise entre la température ambiante et la température de reflux du solvant alcoolique.

5. Un procédé selon la Revendication 4, effectué à une température comprise entre 50°C et 70°C.

6. Un procédé selon l'une des Revendications 1 à 5, dans lequel la résine synthétique à échange de cations est un copolymère d'acide méthacrylique et de divinylbenzène sous la forme d'acide libre.

7. Un procédé selon l'une des Revendications 1 à 6, dans lequel la ranitidine est utilisée sous la forme d'une base libre.

8. Un procédé selon l'une des Revendications 1 à 7, dans lequel la résine synthétique à échange de cations et la ranitidine sont utilisées dans des quantités telles que l'absorbate de résine a une teneur en ranitidine allant de 5 % à 70 % par rapport à la masse, le pourcentage étant exprimé en fonction de la masse de base libre de ranitidine.

9. Un procédé selon la Revendication 8, dans lequel la teneur en ranitidine de l'absorbate de résine est de 25 % à 35 %.

10. Un absorbate de résine formé entre la ranitidine et une résine synthétique à échange de cations, ayant une teneur en eau inférieure à 3 % par rapport à la masse.

11. Un absorbate de résine selon la Revendication 10, dans lequel la teneur en eau est inférieure à 1 %.

12. Un absorbate de résine selon la Revendication 10 ou 11, formé entre la ranitidine et une résine d'acide méthacrylique et de divinylbenzène sous la forme d'acide libre.

13. Une composition pharmaceutique destinée à être administrée par voie orale en médecine pour les hommes ou pour les animaux, contenant un absorbate de résine lorsqu'il est préparé par un procédé selon l'une des Revendications 1 à 9.

14. Une composition pharmaceutique destinée à être administrée par voie orale en médecine pour les hommes ou les animaux, contenant un absorbate de résine selon l'une des revendications 10 à 12.

**Revendications pour les Etats contractants suivants : ES, GR**

1. Un procédé de préparation d'absorbate de résine de ranitidine comprenant le fait de mettre une résine synthétique à échange de cations en contact avec de la ranitidine ou un sel de celle-ci acceptable d'un point de vue physiologique, en présence d'un solvant alcoolique.

2. Un procédé selon la Revendication 1, dans lequel la résine synthétique à échange de cations est mélangée avec une solution de ranitidine sous la forme de sa base libre ou d'un sel de celle-ci acceptable d'un point de vue physiologique, dans le solvant alcoolique.

3. Un procédé selon la Revendication 1 ou 2, dans lequel le solvant alcoolique est du méthanol, de l'éthanol ou un mélange de ceux-ci.

4. Un procédé selon l'une des Revendications 1 à 3, effectué à une température comprise entre la température ambiante et la température de reflux du solvant alcoolique.

5. Un procédé selon la Revendication 4, effectué à une température comprise entre 50°C et 70°C.

6. Un procédé selon l'une des Revendications 1 à 5, dans lequel la résine synthétique à échange de cations est un copolymère d'acide méthacrylique et de divinylbenzène sous la forme d'acide libre.

7. Un procédé selon l'une des Revendications 1 à 6, dans lequel la ranitidine est utilisée sous la forme d'une base libre.

8. Un procédé selon l'une des Revendications 1 à 7, dans lequel la résine synthétique à échange de cations et la ranitidine sont utilisées dans des quantités telles que l'absorbate de résine a une teneur en ranitidine allant de 5 % à 70 % par rapport à la masse, le pourcentage étant exprimé en fonction de la masse de base libre de ranitidine.

9. Un procédé selon la Revendication 8, dans lequel la teneur en ranitidine de l'absorbate de résine est de 25 % à 35 %.